(19) Europäisches Patentamt
European Patent Office
Office européen des brevets

(11) **EP 3 628 972 A1**

(12) **EUROPEAN PATENT APPLICATION**

(43) Date of publication:
**01.04.2020   Bulletin 2020/14**

(51) Int Cl.:
**G01C 21/34** (2006.01)         **G01C 21/36** (2006.01)
**G01N 33/00** (2006.01)

(21) Application number: **19169053.6**

(22) Date of filing: **12.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2018   EP 18196996**

(71) Applicant: **Valeo Systemes Thermiques
78320 Le Mesnil Saint Denis (FR)**

(72) Inventors:
• **DE PELSEMAEKER, Georges**
  **78322 Le Mesnil Saint Denis Cedex (FR)**
• **CLEMARON, Laetitia**
  **78322 Le Mesnil Saint Denis Cedex (FR)**
• **DEVEYCX, Marion**
  **78322 Le Mesnil Saint Denis Cedex (FR)**
• **AMORIM, Laurie**
  **78322 Le Mesnil Saint Denis Cedex (FR)**
• **PORTAL, Younick**
  **61430 Athis de L'Orne (FR)**

(74) Representative: **Metz, Gaëlle
Valeo Systèmes Thermiques
8, rue Louis Lormand
CS 80517 La Verrière
78322 Le Mesnil Saint Denis Cedex (FR)**

(54)   **A COMPUTER-IMPLEMENTED METHOD AND A SYSTEM FOR DETERMINING A ROUTE**

(57)   A computer-implemented method for determining a route, in particular for a vehicle, comprising:
- acquiring navigation data, a starting point (S) and a destination point (D) and determining (110) a plurality of routes and the travelling time;
- acquiring (130) air pollution forecast data including air quality indexes;
- acquiring (140) user preference information;
- determining (150) a first route score, based on user preference information, for each of the plurality of routes without taking into account the air pollution forecast data, so that a first ranked group of routes is obtained;
- calculating (160a) exposure factor (E; E_FA) based on the average air quality index and the travelling time for each of said first group of routes;
- determining (160) a second route score, based on user preference information, for each of said first group of routes with taking into account the calculated exposure factor (E; E_FA), so that a second ranked group of routes is obtained;
- selecting and outputting (170) a first (R1) and a second (R2) route candidates.

## Fig.2

|     | Ra   | Rb   | Rc   |
| --- | ---- | ---- | ---- |
| AQI | 45   | 30   | 40   |
| dT  | 60   | 80   | 70   |
| E   | 2700 | 2400 | 2800 |

EP 3 628 972 A1

**Description**

**FIELD OF THE INVENTION**

The invention relates to a determination of a route. In particular, it relates to the determination of a plurality of routes in an environment subject to air pollution. The invention also relates to an associated system for determining a route and computer readable storage medium storing a program of instructions executable by a machine to perform the method.

**BACKGROUND OF THE INVENTION**

Air pollution is a serious concern for people because of its harmful effects to health - both in physical and psychological sense. Passengers travelling in vehicles are especially at risk, as the vehicles are part of traffic existing in urbanized areas, which tend to have higher concentration of air pollution and higher levels of pollutant gases and particles. Naturally, people commuting by foot, by bicycles or motorcycles are also or even more concerned.

Pollution levels are monitored by government agencies and private entities. The measurements are often provided in form of so called pollution maps. For example, a layer of visually represented information is added to a classic map, so that pollution concentration within a specific area or region can be determined and evaluated. Such maps usually have a set time-resolution, which means that the information is updated for example once an hour or once a day. Pollution map data is obtained for example by measuring pollution with a number of measuring stations or pollution measurement devices of a fixed position.

A typical navigation system, for example vehicle navigation system, is configured to propose one or more route candidates between a starting point and selected destination point. The routes are usually ranked based on several criteria, inter alia distance, time or cost.

**SUMMARY OF THE INVENTION**

The object of the invention is, among others, a computer-implemented method for determining a route, in particular for a vehicle, comprising steps of:

- acquiring navigation data, a starting point and a destination point and determining from navigation data a plurality of routes between the starting point and the destination point and the travelling time for each of the plurality of routes;

- acquiring air pollution forecast data including air quality indexes for the plurality of routes;

- acquiring user preference information;

- determining a first route score, based on user preference information, for each of the plurality of routes without taking into account the air pollution forecast data, so that a first ranked group of routes is obtained;

- calculating exposure factor based on the average air quality index and the travelling time for each route of said first group of routes;

- determining a second route score, based on user preference information, for each route of said first group of routes with taking into account the air pollution forecast data and the calculated exposure factor, so that a second ranked group of routes is obtained;

- selecting and outputting a first route candidate and a second route candidate, wherein the first route candidate is the candidate with optimal first route score from said first group of routes and the second route candidate is the candidate with optimal second route score from said second group of routes.

The method may be implemented by a system including an on-board computer of the vehicle and/or an information system source of the vehicle and/or an entertainment system of the vehicle and/or a mobile terminal and/or a user interface and/or a navigation services provider and/or a data supplier and/or cloud computing services, said system including communication means, control means and output means.

Preferably, the steps of acquiring navigation data, a starting point and a destination point and determining a plurality of routes between the starting point and the destination are implemented by the communication means and control means of the system.

For acquiring navigation data, a starting point and a destination point and determining a plurality of routes between the starting point and the destination point, by communication means and control means of the system, for example of a navigation module embedded in the vehicle such as the vehicle entertainment system, and/or through an application on the mobile terminal and/or of the navigation services provider, the method may comprise:

- setting a starting point and a destination point through a user interface embedded in the vehicle or through an application of the mobile terminal, and/or detecting the current position of the user or vehicle by the navigation module,

- transmitting the starting point and destination point to communication means, for instance of the navigation module,

- sending a request with communicating the starting point and destination point from the communication means of the navigation module to a navigation services provider for obtaining navigation data, and/or to the cloud or control means embedded in the vehicle, for example part of the on-board computer of the vehicle, for obtaining the plurality of routes,

- receiving by the communication means the navigation data from the navigation services provider and/or the plurality of routes determined by cloud computing services and/or by control means embedded in the vehicle.

Preferably, the step of acquiring air pollution forecast data including air quality indexes for the plurality of routes is implemented by the communication means of the system.

Preferably, the air pollution forecast data is at least partly obtained from a pollution map from a data supplier.

Preferably, the air pollution forecast data is at least partly obtained from the vehicle's onboard pollution sensors.

In one option, for acquiring air pollution forecast data including air quality indexes for the plurality of routes by communication means of the system, for example of the vehicle's on-board computer and/or of the data supplier of air pollution forecast data and/or from vehicle's onboard pollution sensors, the method may comprise:

- sending a request by the communication means for obtaining air pollution forecast data to a data supplier of air pollution forecast data with communicating a specified area and/or to pollution sensing means mounted on the vehicle for obtaining real-time pollution measurement data,

- receiving by the communication means air pollution forecast data from the data supplier and/or the real-time pollution measurement data from the sensing means,

- transmitting by the communication means the acquired air pollution forecast data and/or the real-time pollution measurement data to the control means.

Preferably, the step of acquiring user preference information is implemented by the communication means and/or control means of the system.

In one option, for acquiring user preference information by the communication means and control means of the system, for example of the vehicle's entertainment system or through an application on the mobile terminal, the method may comprise:

- receiving by the communication means and/or the control means, for example of the vehicle's entertainment system or through an application on the mobile terminal, the user preference information from input means, for instance a user interface part of the vehicle entertainment system or of the mobile terminal such as a touch screen,

- possibly transmitting by the communication means the preference information to the control means of the system, embedded in the vehicle, for example part of the on-board computer of the vehicle, or through the application of the smartphone or cloud computing services.

Preferably, the step of determining a first route score, based on user preference information, for each of the plurality of routes without taking into account the air pollution forecast data, so that a first ranked group of routes is obtained, is implemented by the communication means and control means of the system.

Preferably, the step of calculating exposure factor based on the average air quality index and the travelling time for each route of said first group of routes, is implemented by the control means of the system.

The method may comprise acquiring air quality indexes at several measuring points for each route, then calculating the average air quality index for each route.

Preferably, the step of determining a second route score, based on user preference information, for each route of said first group of routes with taking into account the air pollution forecast data and the calculated exposure factor, so that a second ranked group of routes is obtained, is implemented by the control means of the system.

Preferably, the step of selecting a first route candidate and a second route candidate is implemented by the control means of the system.

Preferably, the step of outputting a first route candidate and a second route candidate is implemented by output means. The output means may or may not be the same as input means.

Outputting a first route candidate and a second route candidate may be by displaying the first route candidate and a second route candidate on a user interface like a screen or a touch screen of a mobile terminal and/or of the vehicle's entertainment system. The output means may be a user interface like a screen or a touch screen of a mobile terminal and/or of the vehicle's entertainment system.

In one option, the exposure factor is calculated applying the formula (a): $E = AQI * dT$, with $AQI$ being the average air quality index, $dT$ being the traveling time in minutes.

The first route candidate with the optimal first route score may be the route candidate with the minimal traveling time or the minimal traveling distance or the minimal cost.

In one option, the second route candidate with optimal second route score is the candidate route with the minimal exposure factor from the second group of routes.

Preferably, the method comprises the step of calculating exposure time with average air quality index less than or equal to a first threshold for each of the first group of routes.

The step of determining a second route score for each of the first group of routes may be implemented with taking into account the exposure time with average air quality index less than or equal to the first threshold calculated, so that said second group of routes is obtained. The first threshold is for instance 25.

The second route candidate with optimal second route score may be the candidate route with the longest exposure time with average air quality index less than or equal to the first threshold from said second group of routes.

The method may comprise calculating exposure time with average air quality index greater than a second threshold for each of the plurality of routes and/or calculating exposure time with average air quality index greater than the first threshold and less than or equal to the second threshold for each of the plurality of routes. The second threshold is for instance 50 or 75.

The step of determining a second route score for each of said first group of routes may be implemented with taking into account the exposure time with average air quality index greater than the second threshold and/or the exposure time with average air quality index greater than the first threshold and less than or equal to the second threshold calculated, so that said second group of routes is obtained.

The second route candidate with optimal second route score may be the candidate route with no or the least exposure time with average air quality index greater than the second threshold.

Preferably, the step of calculating exposure factor is based on the average air quality index and the travelling time when a heating and/or ventilating and/or air conditioning installation of the vehicle operates in a fresh air mode for each of said first group of routes, applying the formula (b): $E\_FA = AQI\_FA * dT\_FA$, with $AQI\_FA$ being the average air quality index when said installation operates in the fresh air mode, $dT\_FA$ being the traveling time when said installation operates in the fresh air mode in minutes.

In one option, the method may comprise a step of estimating the clogging level of a filter of a heating and/or ventilating and/or air conditioning installation of the vehicle.

The step of determining a second route score for each of said first group of routes may be implemented with taking into account the estimated filter clogging level.

In one option, said installation includes a recirculating flap mobile between an open position and a closed position to operate between a fresh air mode and a re-circulating mode, and the method comprises, for estimating the filter clogging level, following steps of:

- acquiring vehicle's parameters, such as average air mass flow and recirculating time based on calculation or the average based on learning,

- acquiring filter parameters data, such as air filter efficiency,

- estimating closing or opening area of recirculating flap based on external pollution and internal carbon dioxyde and humidity levels, if possible, otherwise on the average external pollution,

- acquiring pollution levels preferably when recirculating flap is open, i.e. while operating in fresh air mode,

- estimating weight of the filter using the obtained filter parameters data, the air pollution forecast data,

- matching the estimated weight with a predefined clogging level, and

- outputting the air filter clogging level estimation.

The second route candidate with optimal second route score may be the candidate route with the least filter clogging.

For example, the routes of said first group of routes can be sorted according to the efficiency of the filter and then according to the exposure factor, the exposure factor in fresh air mode, the exposure time with a low AQI, etc...

In one option, the method comprises a step of obtaining weather forecast data for the plurality of routes. The step of determining a first route score for each of the plurality of routes may be implemented based on user preference information, without taking into account the weather forecast data and the step of determining a second route score for each of said first group of routes may be implemented based on user preference information, with taking into account the weather forecast data.

Preferably, the air pollution forecast data is at least partly obtained from a pollution map from a data supplier and/or from vehicle's onboard pollution sensors.

Preferably, the air pollution forecast data and/or weather forecast data is at least partly obtained from the vehicle's system information source comprising at least one from the group of: wipers speed, lighting operation, braking efficiency.

Preferably, the air pollution forecast data and/or weather forecast data is at least partly obtained from at least one from the group of: meteorological stations, pollution stations with a fixed location, a personal mobile pollution sensors (pollution pods), other vehicles, a satellite, a weather map.

Preferably, the method is iterated based on elapsed time or based on a travelled distance.

Preferably, the method is iterated for each update of air pollution forecast data.

Preferably, the first and/or second route score is determined based on at least one criterion selected from the group: distance, duration, cost.

Preferably, the second route score is calculated based on at least one criterion selected from the group: sun exposure, pollution level, allergen exposure, driving conditions.

Another object of the invention is a system comprising:

- communication means and control means configured to:

    acquire navigation data, starting point and destination point,

    determine a plurality of routes from navigation data between the starting point and destination point and determine the travelling time for each of the plurality of routes,

    acquire air pollution forecast data including air quality indexes for the plurality of routes,

    acquire user preference information;

    determine a first route score, based on user preference information, for each of the plurality of routes without taking into account the air pollution forecast data so that a first ranked group of routes is obtained; and

    calculate the average air quality index for each route of the first group of routes, and the exposure factor based on the average air quality index and the travelling time for each route of the first group of routes,

    determine a second route score, based on user preference information, for each route of the first group of routes with taking into account the air pollution forecast data and the calculated exposure factor so that a second ranked group of routes is obtained,

    select a first route candidate and a second route candidate, wherein the first route candidate is the candidate with optimal route score from said first group of routes and the second route candidate is the candidate with optimal route score from said second group of routes;

- output means configured to output the first route candidate and the second route candidate.

The system may include an on-board computer of the vehicle and/or an information system source of the vehicle and/or an entertainment system of the vehicle and/or a mobile terminal and/or a navigation services provider and/or a data supplier and/or cloud computing services.

Preferably, the system further comprises pollution sensing means adapted to be mounted on a vehicle, configured to provide real-time pollution measurement data, indicative of pollution levels within the vehicle cabin and/or outside the vehicle's cabin, in its immediate vicinity.

Preferably, the communication means are configured to obtain the air pollution forecast data. As an alternative or in addition, the communication means can also be configured to obtain weather forecast data at least partly from the vehicle's system information source comprising at least one from the group of: wipers speed, lighting operation, braking efficiency.

Another object of the invention is a computer readable storage medium storing a program of instructions executable by a machine to perform a method as described above.

**BRIEF DESCRITPTION OF DRAWINGS**

Examples of the invention will be apparent from and described in detail with reference to the accompanying drawings, in which:

    Fig. 1 shows a flowchart presenting the method according to the invention;

    Fig. 2 shows a table of results of exposure factor during the raveling time for three example routes;

    Fig. 3 shows a table of results of exposure time with a low air quality index and with a high air quality index for three example routes;

    Fig. 4 shows a table of results of exposure factor in a fresh air mode of the vehicle for three example routes;

    Fig. 5 schematically shows a graph of routes with segments of different pollution level and depending on whether the vehicle operates in a fresh air mode or a re-circulating air mode;

    Fig. 6 is a table of results of air filter efficiency for the different segments of a first route of routes of Fig. 5;

    Fig. 7 is a table of results of air filter efficiency for the different segments of a second route of routes of Fig. 5;

    Fig. 8 is a table of results of air filter efficiency for the different segments of a third route of routes of Fig. 5;

    Fig. 9 shows an example of outputted route candidates;

Fig. 10 shows a schematic representation of a system according to invention.

## DETAILED DESCRIPTION OF EMBODIMENTS

The following embodiments are examples. Although the description refers to one or more embodiments, this does not necessarily mean that each reference relates to the same embodiment, or that the features apply only to a single embodiment. Simple features of different embodiments may also be combined or interchanged to provide other embodiments.

In the description, it is possible to index some elements, such as for example first element or second element. In this case, it is a simple indexing to differentiate and name close but not identical elements. This indexing does not imply a priority of one element with respect to another and such denominations can be interchanged without departing from the scope of the present description. This indexing does not imply any order in time.

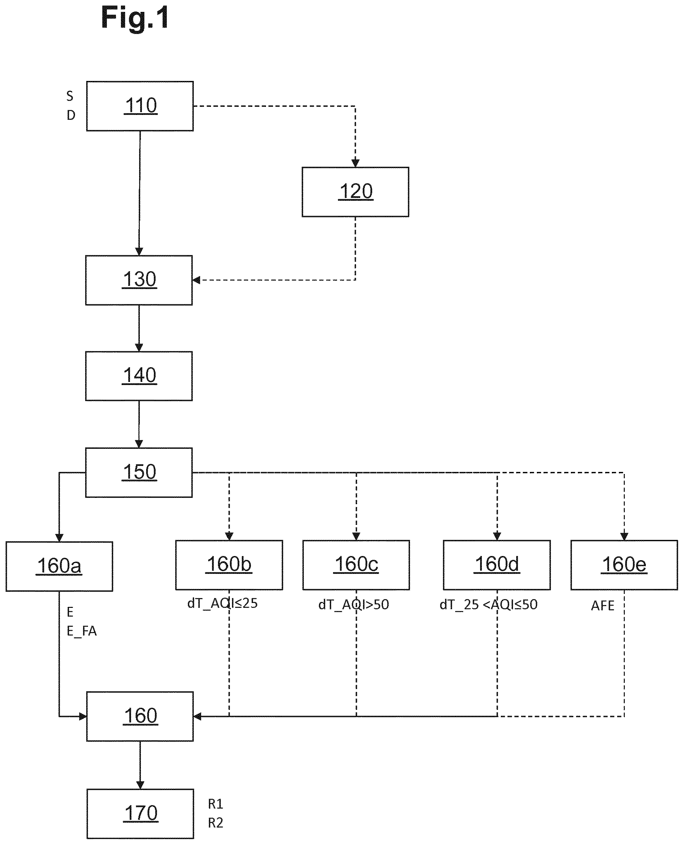

Fig. 1 shows a flowchart presenting the method according to the invention for determining a route, in particular for a vehicle. Firstly, the method comprises determining 110 a plurality of routes between a starting point S and a destination point D. For this purpose navigation data, starting point S and destination point D are acquired or obtained.

The starting point S can be input manually by the user through a user interface or can be obtained from the navigation data automatically, by known means. The user can set the destination point D using a navigation module embedded in the vehicle or through an application of a mobile terminal like a smartphone.

Next, the method can comprise a step of obtaining 120 weather forecast data for the plurality of routes.

The weather forecast data concerns most recent weather conditions and forecasted weather conditions. It can include temperature, barometric pressure, wind speed, sun exposure, humidity, visibility, time of sunrise and sunset, ultraviolet index etc. Information on current and forecasted weather conditions may be taken into account in view of its influence on safety and/or attractiveness to the user. Some weather conditions may imply worse driving conditions, which in turn imply worse safety.

In one option, the weather forecast data is at least partly obtained from the vehicle's system information source comprising at least one from the group of: wipers speed, lighting operation, braking efficiency. In other words, the data is obtained in an indirect manner from the vehicle on-board information system. For example, a currently set wipers speed may be indicative of raining intensity. The set lightening parameters may be indicative of visibility in front of the vehicle. The measured braking efficiency may be indicative of road conditions, and for example be correlated with rain, ground frost etc.

The method comprises a step of acquiring or obtaining 130 air pollution forecast data for the plurality of routes.

The air pollution forecast data concerns most recent pollution levels and forecasted pollution levels. More precisely, outside pollution data, indicative of pollution level outside the vehicle's cabin, can be acquired. Preferably, it is at least partly acquired or obtained from a pollution map from a data supplier. Such pollution map data can comprise information on particulate matter levels, carbon monoxide, sulfur oxides, nitrogen oxides, ozone, spread over a defined geographical area. It is also envisaged that this pollution map data may be supplemented with information on pollens and allergens. Pollution data also relate to air quality indexes for the defined geographical area.

Additionally, the air pollution forecast data may be at least partly obtained from the vehicle's onboard pollution sensors. In particular, these sensors can output data indicative of pollution levels outside the vehicle cabin, in the immediate vicinity of the vehicle. The data can be then used for real-time adjustment of the forecast, e.g. using machine learning models. The data obtained by measurements from the vehicle's onboard pollution sensors might also be sent to a cloud and then obtained by other users.

In one option, the air pollution forecast data and/or weather forecast data is at least partly obtained from at least one from the group of: meteorological stations, pollution stations with a fixed location, a personal mobile pollution sensors (pollution pods), other vehicles, a satellite, an open source pollution or weather maps.

In the next step, the method comprises a step of obtaining 140 user preference information. The user preference information may relate for example to the distance, duration or a cost of the trip. These preferences may be grouped into a first group of preferences.

The user preference information may relate for example also to pollution levels such as air quality indexes and weather. These preferences may be grouped into a second group of preferences. In particular, the second group of preferences may relate to sun exposure, pollution level, allergen exposure, safety (driving conditions). The second group of preferences may also relate to additional time allowed with respect to a fastest route candidate for example to be determined based on the first group of preferences.

The pollution level can be the outside pollution level and/or the pollution level inside the vehicle's cabin.

For example, the user may input preference information indicating preference of a route with maximized sun exposure, with limited cloud coverage of the sky, with minimized pollution level. Alternatively, the user may input preference information indicating preference of a route with good driving conditions and limited sun exposure.

For another example, the user may input preference information relating to the air quality index, such as preferences to avoid routes with a long exposure to high pollution levels, namely with high air quality index, for instance greater than 50 or 75, and/or to whether consider the average air quality index during the whole travelling time or when operating in fresh air mode in the case of a vehicle comprising a heating and/or ventilating and/or air conditioning installation able to operate in a fresh air mode. Said installation generally includes a re-circulation flap also named recycling flap mobile between an open position and a closed position to operate between the fresh air mode and a re-circulating mode.

Alternatively or in addition, in order to prevent or limit exposure of vehicle passengers to polluted air, said installation generally comprises one or more filters for the air which is introduced into the cabin. The user may input preferences information about the efficiency of such air filter of said installation, for instance indicating preference of a route that limits the clogging of the air filter and/or degrades the least the air filter performance.

It is also envisaged that a third group of preferences may be formed, which could relate to attractiveness of the route in view of preferred sport activities, points of interest exposure, nature/urban ratio exposure, type of terrain etc.

In the next step, the method comprises a step of determining 150 a first route score, based on user preference information, for each of the plurality of routes without taking into account the weather forecast data and the air pollution forecast data, so that a first group of routes is obtained. In other words, a first route score is determined for each of the plurality of routes only based on the first group of preferences. What follows is that, preferably, the first route score is determined based on at least one criterion selected from the group: distance, duration, cost, in a known manner. The method may comprise a step of ranking the routes based on their determined first route scores, so that the first group of routes is obtained.

In a next step, the method comprises a step of determining 160 a second route score, based on user preference information, for each route of the first group of routes with taking into account the air pollution forecast data, in particular the calculated exposure factor E; E_FA, and possibly the weather forecast data, so that a second group of routes is obtained. In other words, a second route score is determined for each of the plurality of routes based on the first group of preferences and the second group preferences. The method may comprise a step of ranking the routes based on their determined second route scores, so that the second group of routes is obtained.

The first group of routes is not necessarily all obtained before implementing the step 160 of determining the second route scores. The steps 150, 160 of determining route scores can be implemented at least partly at the same time.

For obtaining the second route candidate R2, the air quality index, and possibly a weather index or others can be calculated for each measuring point, then combined together to form scores for each measuring point and then the average score is calculated for each route.

For that purpose, according to a preferred embodiment, the method comprises at least a step of calculating 160a an exposure factor E; E_FA based on the average air quality index and the travelling time dT; dT_FA for each route of the first group of routes.

The exposure factor E may be calculated for the whole traveling time. As an alternative, for a vehicle comprising a heating and/or ventilating and/or air conditioning installation able to operate in a fresh air mode, the exposure factor E_FA may be calculated based on the average air quality index and the travelling time dT_FA when said installation operates in the fresh air mode for each route of the first group of routes.

What follows is that, preferably, the route score may be additionally determined based on at least one criterion selected from the group: sun exposure, pollution level, allergen exposure, safety (driving conditions).

In one option, the method further comprises a step of calculating 160b exposure time with air quality index less than or equal to a first threshold, for instance 25, for each route of the first group of routes. The first threshold can be predetermined, set by the user, or advised by world health organization.

The method can further comprise a step of calculating 160c exposure time with air quality index greater than a second threshold, for instance 50 or 75, for each route of the first group of routes. The second threshold can be predetermined, set by the user, or advised by world health organization. The second threshold can also be set depending on the sensitivity of the user and/or passengers in the vehicle.

The method can also comprise a step of calculating 160d exposure time with air quality index greater than the first threshold and less than or equal to the second threshold for each route of the first group of routes.

In another option, for said installation including an air filter, the method may comprise a step of estimating 160e the air filter clogging level for each route of the first group of routes.

These embodiments relating to the steps 160a to 160e are described in more detail in the following description.

Thus, the step of determining 160 a second route score can be implemented with also taking into account the exposure time with average air quality index less than or equal to the first threshold, and/or avoiding routes exposed to high air quality indexes, for instance greater than 50 or 75, and/or taking into account the clogging and the efficiency of the air filter of said installation. In other words, the step of determining 160 a second route score can be implemented using the information determined and collected after implementing one or more of the steps 160a to 160e.

Alternatively or additionally to steps 160a to 160e, the second route candidate R2 is a compromise between the best score and additional time with respect to the first route candidate R1. It is envisaged that the second route candidate is constrained to add for example 10% of travelling time for short trips which do not exceed 30 minutes, 5% for trips between 30min and 2h and then 2% for longer trips. In other words, the second route candidate R2 is constrained to a specific range of additional time with respect to the first route candidate R1.

Analogously, if a third group of preferences is present, the method can comprise determining a third route score for each route of the plurality of routes based on the first group of preferences, the second group preferences and the third group of preferences, so that a third group of routes is obtained.

In the next step, the method comprises selecting and outputting 170 a first route candidate R1 and a second route candidate R2. This outputting can be done through displaying on the user interface, such as a touch screen of the vehicle's on-board computer and/or of a portable communication device or mobile terminal as a smartphone.

The first route candidate R1 is the candidate route with optimal route score from the first group of routes and the second route candidate R2 is the candidate with optimal route score from the second group of routes. The determination of the optimal candidate in both cases can be carried out by known optimal route determination algorithms. The optimal route score in both cases may be for example that which corresponds most closely to the selected preference information parameters. The selection of the second route candidate R2 in any case needs to take into account more parameters than the first route candidate R1. The first route candidate R1 and the second route candidate R2 may or may not be the same.

For instance, the first route candidate with the optimal first route score may be the route candidate with the minimal traveling time or the minimal traveling distance or the minimal cost.

As non-limitative examples, the second route candidate with optimal second route score may be the candidate route from the second group of routes with the minimal exposure factor, or with the longest exposure time with average air quality index less than or equal to the first threshold, with no or the least exposure time with average air quality index greater than the second threshold, or with the least filter clogging.

Analogously, if a third group of preferences is present, the method can comprise selecting and outputting a third route candidate which is the candidate with optimal route score from third group of routes.

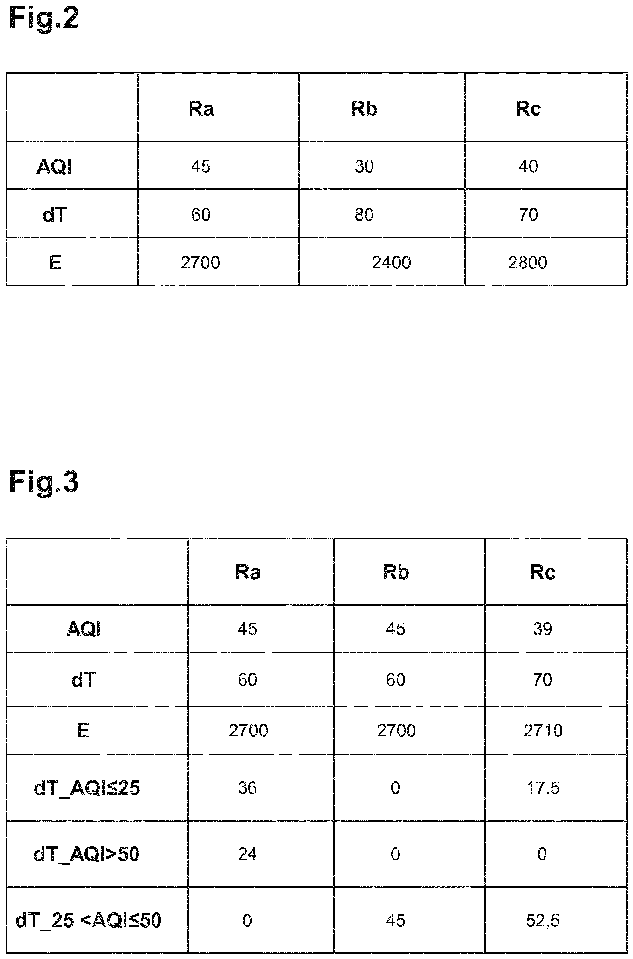

The embodiments relating to the steps 160a to 160e are described hereafter in more detail with reference to figures 2 to 8.

Fig. 2 shows a table of results illustrating the first embodiment according to which the exposure factor E is calculated for the whole traveling time, for each route of the first group of routes.

Three example routes Ra, Rb, Rc are given for illustration in a non-limited way.

For each route Ra, Rb, Rc, air quality indexes can be acquired at several measuring points, then the average air quality index is calculated for each route. The distance between the measuring points can be set depending on the type of road, for instance they can be close or more frequent in a more polluted area such as the city, while farther apart from each other in country side. As a simple example, the measuring points can be every 3meters to 50meters in city or 10meters to 250-500meters in country or every minute.

The whole travelling time is obtained for each route, for example thanks to a navigation module.

In the example given, in a merely illustrative way:

- route Ra is exposed to an average air quality index AQI of 45 for instance, during a total traveling time dT of 60minutes,
- route Rb is exposed to an average air quality index AQI of 30 for instance, during a total traveling time dT of 80minutes,
- route Rc is exposed to an average air quality index AQI of 40 for instance, during a total traveling time dT of 70minutes.

Next, the exposure factor E is calculated for each route applying the formula (a):

$$(a) \ E = AQI * dT;$$

with AQI being the average air quality index and dT being the traveling time in minutes.

As a result, the exposure factor E is 2700 for route Ra, 2400 for route Rb, 2800 for route Rc.

The second route candidate R2 may be the candidate with the minimal exposure factor E. The method thus comprises a step of comparing the calculated exposure factors E each other to determine the lowest. In this example merely illustrative, the route Rb has the lowest exposure factor and can be determined as the second route candidate R2.

Alternatively, the exposure factor can be weighed by the additional time implied by the lower average air quality index. If the user has input preferences information not to exceed a certain threshold percentage of additional travelling time, for instance 5% for trips between 30minutes and 2hours, then the method comprises a step of calculating the additional time compared to the first route candidate R1 and to compare with the threshold percentage.

In the example of Fig. 2, the cleanest route regarding the exposure factor E is route Rb, with additional 20minutes of traveling time, in case of a first route candidate R1 with an hour traveling time for example. The additional 20minutes exceed the 5% allowed in the user preferences for example.

The second route candidate R2 can be a compromise between the best score and additional time. In this case, route Ra is preferred even if the average air quality index is higher than the average air quality index of route Rb, because the route Rb has too long travelling time and the route Rc has the highest exposure factor.

Fig. 3 shows a table of results illustrating a second embodiment according to which the selection of the cleanest route is not just based on the average air quality index, but the exposure factor is weighed by air quality index levels.

For instance, the selection of the second route candidate as the clean route is also based on the exposure time dT_AQI≤25 and/or the number of measuring points with the air quality index below or equal to a first threshold, for instance 25. In other words, the second route candidate is the route candidate exposed the most of time to an air quality index below or equal to 25.

In the example of Fig. 3, in a merely illustrative way, routes Ra and Rb are exposed to an average air quality index AQI of 45 for instance during 60minutes, and route Rc is exposed to an average air quality index AQI of 39 for instance during 70minutes.

The exposure factor E is calculated for each route applying the formula (a) and is 2700 for routes Ra and Rb, and 2710 for route Rc.

The method can comprise a step of comparing the air quality indexes acquired at the different measuring points along each route to the first threshold. Next, the exposure time dT_AQI≤25 while the air quality index is less than or equal to a first threshold, for instance 25, can be calculated.

In the example of Fig. 3, the route Ra has an exposure time dT_AQI≤25 of 36minutes with the air quality index below or equal to the first threshold, the route Rb has no exposure time with the air quality index below or equal to the first threshold, and the route Rc has an exposure time dT_AQI≤25 of 17.5minutes with the air quality index below or equal to the first threshold.

The method can comprise a step of comparing the exposure time dT_AQI≤25 with the air quality index below or equal to the first threshold of each route, and then the route with the longest exposure time dT_AQI≤25 with such a very good air quality index is selected as the clean route. In the example of Fig. 3, the route Ra exposed 36minutes to an average air quality index below or equal to 25 is the second route candidate.

Alternatively or in addition, depending on the user profile and possibly depending on passengers in the vehicle, the user may input preferences information to avoid routes with air quality index above a second threshold, for instance 75 or 50 if there is at least one sensitive person inside the car, e.g. with asthma and/or allergens sensitivity.

The method can comprise a step of comparing the air quality indexes acquired at the different measuring points along each route to the second threshold. Next, the exposure time dT_AQI>50 or the number of measuring points with air quality index greater than the second threshold, for instance 50, can be calculated.

In the example of Fig. 3, the route Ra has an exposure time dT_AQI>50 of 24minutes with the air quality index above the second threshold, the routes Rb and Rc have no exposure time dT_AQI>50 with the air quality index above the second threshold.

The method can comprise a step of comparing the exposure time dT_AQI>50 with the air quality index above the second threshold of each route, and then the route with a long exposure time dT_AQI≤25 with a very good air quality index and with a limited exposure time dT_AQI>50 with a bad air quality index is selected as the clean route. In the example of Fig. 3, the route Rc can be selected as the second route candidate R2, because the route Ra has the longest time with a bad air quality and the route Rb is not exposed to a good air quality.

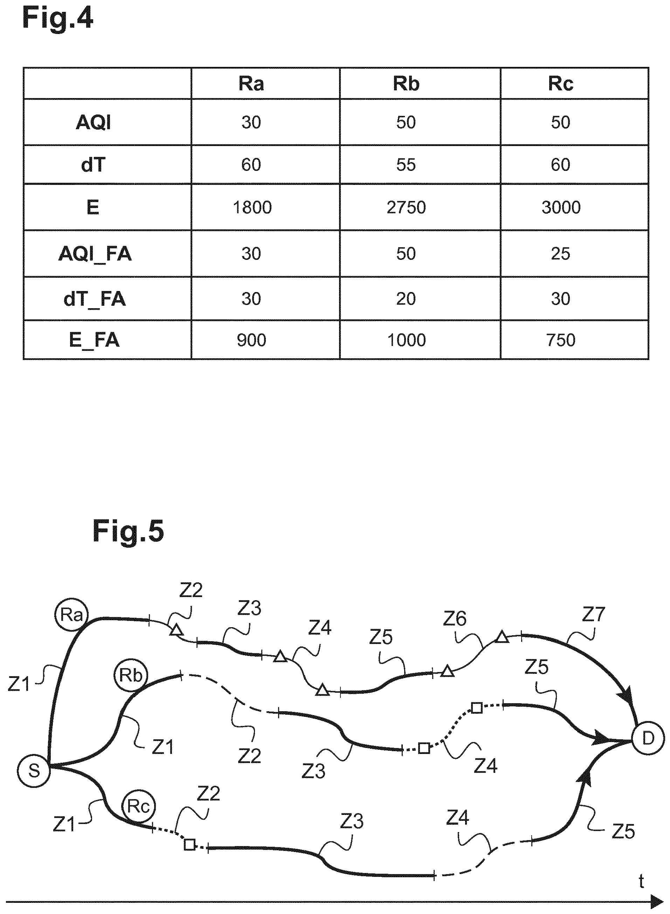

In alternative or in addition, according to a third embodiment, the selection of the second route candidate or clean route can be based on what the user and possible passengers in the vehicle will actually breathe inside the vehicle's cabin. Indeed, the real exposition of the user and passengers of the vehicle depends on the pollution level that enters into the cabin of the vehicle, meaning while fresh air from outside circulates into the cabin, and not necessarily on the entire pollution level outside the vehicle along the whole trip. Fig. 4 shows a table of results illustrating this third embodiment according to which the selection of the clean route is based on the operation of said installation in a fresh air mode.

For that purpose, the method may comprise one or more steps for anticipating for each route of the first group of routes, where said installation will operate in a fresh air mode, meaning with the recycling flap in the open position, along the trip.

Such anticipation or forecasting opening zones of the recycling flap into fresh air mode can be done by any known method.

According to one embodiment, for each route of the first group of routes, less polluted areas can be identified based on the pollution data acquired including the air quality indexes, possibly combined with information from outside the vehicle or weather forecast data. In these identified areas, the re-circulation flap will be switched in the open position for operating in the fresh air mode.

Otherwise, in case the air quality inside the vehicle cabin is better than at least one predetermined threshold, the re-circulation flap will be switched in the closed position for operating in the re-circulation mode.

The air quality inside the vehicle can be determined thanks to many data, such as number of passenger, temperature target inside the car, carbon dioxide concentration $CO_2$ level, and humidity level inside the car. In a non-limited way, for that purpose, data relative to initial conditions or parameters inside the vehicle cabin can be measured or acquired, particularly carbon dioxide concentration $CO_2$, temperature, relative humidity inside the vehicle cabin. For instance, they can be set equal to the corresponding parameters outside the vehicle cabin. Further, the number of passengers inside the vehicle cabin can be determined for instance by a passenger detection module. Then, the carbon di-oxide concentration $CO_2$, respectively relative humidity, inside the vehicle cabin at any time can be determined based on the value of the initial parameter added up to the ratio of produced $CO_2$ flow, respectively produced water $H_2O$ flow, based on the number of passengers inside the vehicle cabin, to the ventilation flow multiplied by the time. The results can be compared to the at least one threshold limit in order to define where the recycling flap has to switch between open or close position.

Three example routes Ra, Rb, Rc are given for illustration in a non-limited way. Similarly to the first embodiment, for each route Ra, Rb, Rc, air quality indexes can be acquired at several measuring points, then the average air quality index is calculated for each route. Further, the whole travelling time is obtained, for example from the navigation device.

In the example given, in a merely illustrative way:

- route Ra is exposed to an average air quality index AQI of 30 for instance, with a total traveling time dT of 60minutes,
- route Rb is exposed to an average air quality index AQI of 50 for instance, with a total traveling time dT of 55minutes,
- route Rc is exposed to an average air quality index AQI of 50 for instance, with a total traveling time dT of 60minutes.

Considering the pollution along the whole trip, the exposure factor E calculated for each route applying the formula (a) would be 1800 for route Ra, 2750 for route Rb, 3000 for route Rc.

According to the first embodiment, the second route candidate R2 would be the candidate with the minimal exposure factor E, that is to say route Ra.

Alternatively, the third embodiment only takes into account the pollution level that will enter into the cabin, when said installation will operate in a fresh air mode according to the forecasting opening zones of the recycling flap into fresh air mode. In the example given in a merely illustrative way:

- route Ra is exposed to an average air quality index in fresh air mode AQI_FA of 30 for instance, during 30minutes,
- route Rb is exposed to an average air quality index in fresh air mode AQI_FA of 50 for instance, during 20minutes,
- route Rc is exposed to an average air quality index in fresh air mode AQI_FA of 25 for instance, during 30minutes.

Next, the exposure factor E_FA in the fresh air mode is calculated for each route applying the following formula (b):

$$(b) \ E\_FA = AQI\_FA * dT\_FA;$$

with AQI_FA being the average air quality index when said installation operates in the fresh air mode, dT_FA being the traveling time when said installation operates in the fresh air mode in minutes.

As a result, the exposure factor E_FA in the fresh air mode is 900 for route Ra, 1000 for route Rb, 750 for route Rc.

The second route candidate R2 may be the candidate with the minimal exposure factor E_FA in the fresh air mode. The method thus comprises a step of comparing the calculated exposure factors E_FA in the fresh air mode each other to determine the lowest. In this example merely illustrative, the route Rc has the lowest exposure factor E_FA in the fresh air mode and can be selected as the second route candidate.

As an alternative or in addition to any previous embodiment, according to yet another embodiment regarding figures 5 to 8, the air filter clogging level $C_{filter}$ and/or the efficiency of the air filter AFE depending on seen pollutants along each route can be taken into account.

On the sketch illustrated on Fig. 5, three example routes Ra, Rb, Rc are shown in a non-limited way. Each route is segmented depending on parameters such as the operating mode, i.e. fresh air mode or re-circulating mode, and the outside pollution level, namely the air quality indexes.

In this example, route Ra is segmented into seven zones Z1 to Z7, route Rb is segmented into five zones Z1 to Z5, and route Rc is segmented into five zones Z1 to Z5.

On Fig. 5, when said installation operates in a re-circulating mode, the segments of the routes are drawn in solid lines, when said installation operates in a fresh air mode the segments are differentiated depending on the air quality index for instance. For good air quality indexes, for instance below or equal to 25, the segments are drawn with dashed lines. For medium air quality indexes, for instance between 25 and 50 included, the segments are drawn in solid lines with triangle symbols. For bad air quality indexes, for instance above 50 or 75, the segments are drawn in dotted lines with square symbols.

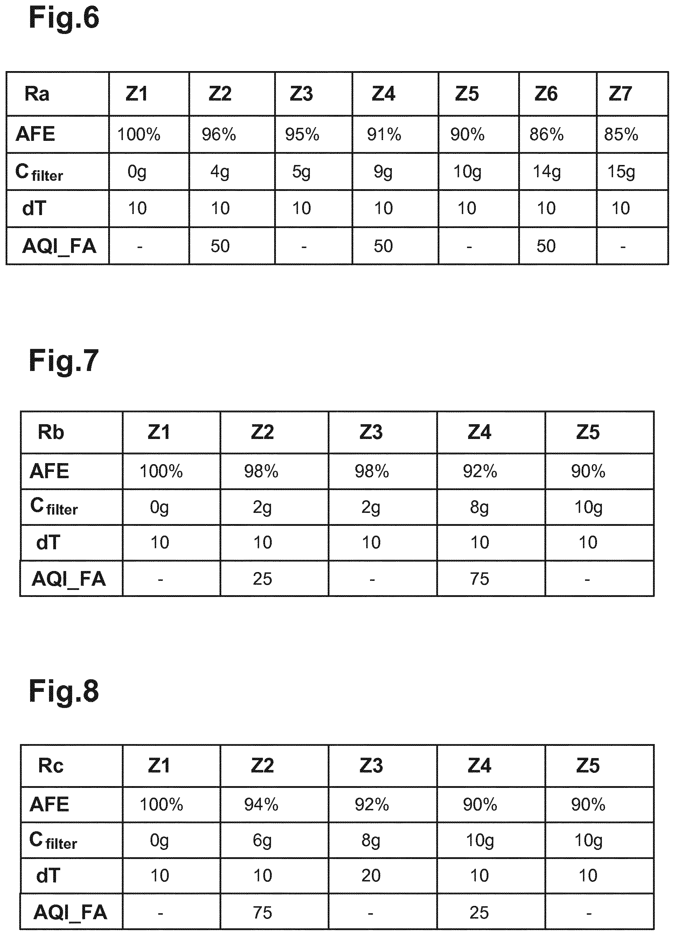

Fig. 6 shows a table of results of the air filter clogging level $C_{filter}$ and the air filter efficiency AFE for the route Ra depending on the pollutants seen on the different zones Z1 to Z7 along the trip.

Fig. 7 shows a table of results of the air filter clogging level $C_{filter}$ and the air filter efficiency AFE for the route Rb depending on the pollutants seen on the different zones Z1 to Z5 along the trip.

Fig. 8 shows a table of results of the air filter clogging level $C_{filter}$ and the air filter efficiency AFE for the route Rc depending on the pollutants seen on the different zones Z1 to Z5 along the trip.

For each route Ra, Rb, Rc, the estimation of the air filter clogging level $C_{filter}$ (figures 6 to 8) can be calculated according to any known manner.

As an example, for estimating the air filter clogging level $C_{filter}$, the method may comprise a step of obtaining or acquiring vehicle parameters data, indicative of the vehicle operation. In particular, the vehicle parameters data comprises parameters of one air quality system of the vehicle. The air filter is generally part of the vehicle air quality system of the vehicle. The vehicle air quality system parameters may be selected from a group comprising: air mass flow through said installation, filter parameters data, recirculation rate or recirculating time of air mass flow or separation of the cabin.

The recirculation rate or time can be based on calculation or the average based on learning.

By separation of the cabin it is meant the ratio between pollutants entering the cabin and pollutants leaving the cabin back into the recycling air flow.

The air filter parameters may comprise filter separation efficiency or air filter efficiency AFE and/or active area of the air filter. In other words, the filter separation efficiency and the active area of the filter may be combined into a single parameter.

The method may further comprise a step of estimating the closing or opening area of recirculating flap, for instance as previously described in reference to the third embodiment. This can be based on external pollution and internal carbon dioxide and humidity levels, if possible, otherwise on the average external pollution.

In the next step, the method may comprise estimating weight of the air filter using obtained data, in particular using the vehicle's parameters data such as the air mass flow through said installation, the recirculation rate of air mass flow, the air filter parameters data like the air filter separation efficiency, the active area of filter, the concentration/ separation of the cabin, and using the air pollution forecast data such as the pollution level outside, preferably when the recirculating flap is in the open position, i.e. while said installation operates in the fresh air mode, the pollution data being either measured by sensor or obtained/acquired from pollution data provider and/or using the pollution level inside the cabin, for example measured by a vehicle on-board sensor.

As previously stated, the pollution data relate to air quality indexes. They may as well relate to particulate matters levels of different sizes PM1, PM2.5, PM10, to emissions of nitrogen oxide NOx, carbon monoxide CO, carbon dioxide $CO_2$, sulfur dioxide $SO_2$, Ozone, Volatile Organic Compounds VOC, allergens and others.

The estimation function can associate the clogging of the air filter with the weight of the air filter. Thus, the estimated weight of the filter will be indicative of its clogging level.

If the amount of collected data is not sufficient, the estimation of the air filter clogging level can be based on kilometers travelled since last filter replacement or first installation, the number of days since last filter replacement or first installation, and the pollution level outside, either measured by a sensor mounted on the vehicle or obtained from pollution data provider, or both.

The coefficients necessary to estimate filter weight based on obtained data such as the abovementioned parameters, can be evaluated and updated during a step of calibrating the system, in which the estimated filter's weight is compared with a measured filter's weight.

In general, the estimation of the air filter clogging level $C_{filter}$ can be calculated according to the following formula (c):

$$(c) : C_{filter} = \sum_i \alpha_i . X_i + \sum_{i,j} \beta_{i,j} . X_i . X_j$$

where $Xi$ and $Xj$ are above described parameters, while $\alpha i$ and $\beta ij$ are identified by filter weighing.

Then, the method may comprise a step of matching the estimated weight with a predefined clogging level. The clogging levels can be stored in the system and be associated with a determined weight, for example with particular weight ranges.

In the next step, the routes can be sorted according to the efficiency of the air filter and/or the clogging level estimation and then according to the exposure factor, the additional traveling time, the exposure factor in fresh air mode, the exposure time with a low air quality index, or any previous embodiment.

The method thus comprises a step of comparing the estimated air filter clogging $C_{filter}$ and/or the air filter efficiency AFE of the routes Ra, Rb, Rc to determine the lowest.

In terms of filter lifetime and efficiency of the air filter, routes Rb and Rc with 10g of filter clogging $C_{filter}$ and 90% of air filter efficiency AFE are better than route Ra with 15g of filter clogging $C_{filter}$ and 85% of air filter efficiency AFE.

In the example given on Fig. 7, in a merely illustrative way, for zones Z1, Z3, Z5 of route Rb, said installation may operate in the re-circulating mode, while operating in a fresh air mode for zones Z2, Z4.

The average air quality index can be calculated for the whole travelling time as explained above regarding the first embodiment or while operating in a fresh air mode as described above regarding the third embodiment.

In the case the average air quality index is calculated in the fresh air mode, for route Rb, the exposure factor in the fresh air mode is calculated for zones Z2, Z4.

For instance, the average air quality index is 25 for zone Z2 during 10minutes, and is 75 for zone Z4 during 10 minutes. Thus, the exposure factor in the fresh air applying the formula (b) is 1000 for route Rb.

In the example given on Fig. 8, in a merely illustrative way, for zones Z1, Z3, Z5 of route Rc, said installation may operate in the re-circulating mode, while operating in a fresh air mode for zones Z2, Z4.

The average air quality index can be calculated for the whole travelling time as explained above regarding the first embodiment or while operating in a fresh air mode as described above regarding the third embodiment.

In the case the average air quality index is calculated in the fresh air mode, for route Rc, the exposure factor in the fresh air mode is calculated for zones Z2, Z4.

For instance, the average air quality index for zone Z2 is 75 during 1 0minutes, whereas zone Z4 has an average air quality index of 25 during 10min. Thus, the exposure factor in the fresh air applying the formula (b) is 1000 for route Rc.

The method thus comprises a step of comparing the calculated exposure factors in the fresh air mode of the routes Rb, Rc to determine the lowest.

In this example, both routes Rb and Rc have the same exposure factor in the fresh air mode. The routes Rb and Rc can be sorted according to another criterion as previously described, for instance depending on the quality of the air, namely as described in the second embodiment on how many times the average air quality index is below or equal to 25 and possibly how many times it is above 75 or 50, or depending on the travelling time.

Thus in this case, combining the travelling time 50minutes for route Rb and 60minutes for route Rc, the exposure time with a good air quality, and the air filter lifetime, the second route candidate is route Rb.

Further, in one option, the method may comprise generating control signals for subsequent operation of the recycling flap in the fresh air mode or in the re-circulating mode when the vehicle reaches corresponding locations. For instance if the user chooses to travel following the outputted second route candidate R2, which is route Rb in the previous example, the control signals may be for operation of the recycling flap in the fresh air mode when the vehicle reaches areas Z2, Z4 and for operation of the recycling flap in the re-circulating mode when the vehicle reaches areas Z1, Z3, Z5.

In alternative or in addition, the method may further comprise outputting the filter clogging level. The outputting can be carried out directly into a vehicle air quality system so that it can adapt its operation parameters. Further or alternatively, the clogging level can be outputted to visual and/or sound indication components of the vehicle or any other alerting signaling means, so that the vehicle user can be informed about the need to replace the filter.

The order of at least part of the steps of the method can be reversed without departing from the scope of the present description. Some steps may for example be performed at the same time.

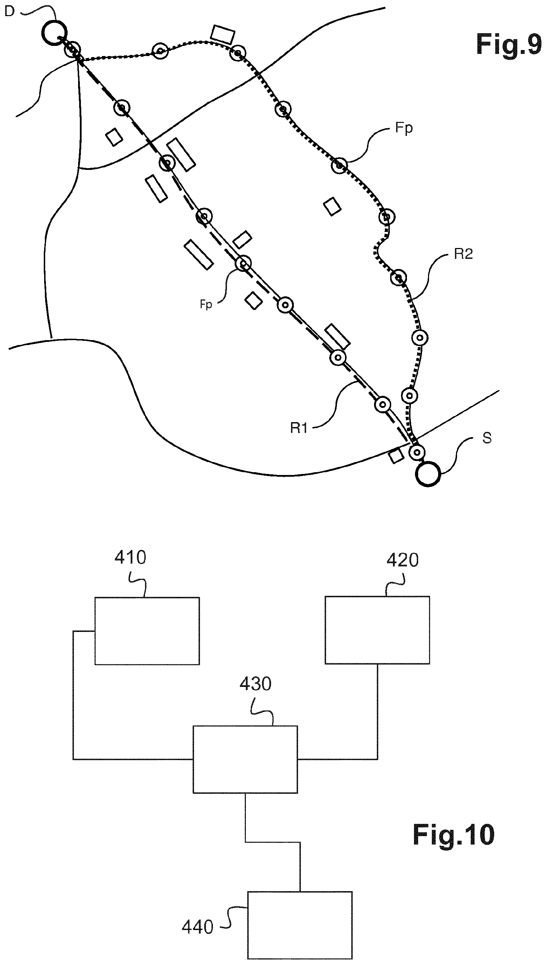

Preferably, part of the steps or all the steps can be iterated based on elapsed time or based on a travelled distance. Alternatively or additionally, the method can be iterated for each update of air pollution forecast data. The iterations are explained in relation to Fig. 9.

Fig. 9 shows an example of outputted route candidates R1 and R2. For that purpose, a starting point S and a destination point D are obtained. In this case, the starting point S can be the current geographical position of the vehicle. A plurality of routes is available between the starting point S and the destination point D. It is shown in Fig. 9 that a first route candidate R1 and a second route candidate R2 are outputted. The first route candidate R1 is shorter and/or could take less time to travel between starting S and destination D points. However, based on the additional second group of preferences, the second route candidate R2 is outputted together with the first route candidate R1. In this case, the second route candidate R2 is different from the first route candidate R1. The user can thus choose between the route candidates, while having more insight about the consequences of the choice. Alternatively, the second route candidate R2 and the first route candidate R1 can be the same.

In Fig. 9 the frequency of iterations of the method or its selected steps along the routes R1, R2 can be observed - the occurrences of iterations are visualised by means of update points Fp. When the vehicle reaches these points along the routes R1, R2, an iteration of the method or at least selected steps takes place and the updated data is obtained. In particular, the updated pollution and/or weather forecast data can be obtained when the vehicle is at update points Fp to propose any new route candidates, which take into account new information. The iteration, as explained above, can be based preferably on air pollution forecast data update rate. However, the distribution of update points Fp can be also based on elapsed time, e.g. once every minute for trips calculated for less than 1 hour and once every ten minutes for longer trips. The distribution of update points Fp can be also based on a travelled distance or traffic information update from the navigation service provider or other basis.

For obtaining the second route candidate R2, the air quality index, possibly a weather index or others can be calculated for each point Fp, then combined together to form scores for each point and then the average score is calculated for each route. At least, the air quality index can be acquired for each point Fp, then the average air quality index is calculated.

Next, the route with the best or optimal score is selected as the second route candidate R2 as previously described.

The above-described method can be carried out by means of cloud computing. In particular, at least part of the steps can be carried out in the cloud, with transmitting the route candidates to the user.

Alternatively or in addition, the method can be carried out by means of on-board computer of the vehicle and/or an information system source of the vehicle and/or an entertainment system of the vehicle. In particular, at least part of the steps can be carried out through the on-board means of the vehicle, with transmitting information, such as the desired destination, the user preferences, pollution levels measurements and possible information of the vehicle operation, to the cloud.

Alternatively or in addition, the method can be carried out by means of any hand held or portable communication device or mobile terminal, such as for example but not limited to laptop, smart phones and the likes. Such portable communication device or mobile terminal can be carried by the vehicle occupants. It may be communicably coupled to the vehicle via a communication network such as for example, but not limited to, WIFI, Bluetooth® and the likes. In particular, at least part of the steps can be carried out through an application of such portable communication device or mobile terminal, with transmitting information, such as the desired destination and the user preferences, to the cloud.

Alternatively or in addition, the method may also be carried out at least partly by means of a remoter server such as a navigation services provider and/or a data supplier of air pollution forecast data like pollution maps, and/or possibly a data supplier of weather forecast data. In particular, the navigation services provider can receive a request for obtaining navigation data between a starting point and a destination point and can transmit the requested navigation data to the cloud and/or on-board means of the vehicle, and/or the mobile terminal. Similarly, the data supplier can receive a request for obtaining pollution forecast data (respectively weather forecast data) between a starting point and a destination point and can transmit the requested data to the cloud and/or on-board means of the vehicle and/or the mobile terminal.

Fig. 10 shows a schematic representation of a system according to the invention configured to carry out at least partly the method previously described.

The system may or may not be entirely embedded in the user's vehicle. In one option, such system may include the on-board computer of the vehicle and/or the information system source of the vehicle and/or the entertainment system of the vehicle and/or the portable communication device or mobile terminal such as a smartphone.

The system comprises communication means 410, computing or control means 430 and output means 440. Additionally, the system can comprise pollution sensing means 420. The system may also comprise a detection module adapted to detect at least one parameter impacting air quality inside the vehicle cabin.

Referring to both figures 9 and 10, the communication means 410 are configured to acquire or obtain navigation data, a starting point S and destination point D and plurality of routes between the starting point S and destination point D. This may be achieved in a conventional manner known in the art, by means of global positioning system (GPS) integration, optionally coupled with internet connection to navigation services provider. Preferably, the communication means 410 are adapted to provide internet connection for the system, in particular to cloud computing services.

The communication means 410 may include one or more transceiver for transmitting and receiving data. The communication means 410 can be adapted to receive and send wireless signals, such as navigation signals, for example GPS signals or the like.

For example, a navigation module can be embedded in the vehicle. This navigation module may or may not be part of the vehicle's entertainment system. Alternatively, the user can have access to a navigation module through an application on the portable communication device or mobile terminal such as a smartphone. The on-board navigation module or through the application of the smartphone includes a global positioning system.

The vehicle's entertainment system generally comprises a user interface, like a touch screen or touch pad, or the user interface may be the screen of the smartphone, through which the user can input starting point S and destination point D. The communication means 410 are configured to receive information from the user interface. The starting point S can also be determined as the current position of the user or vehicle by the navigation module.

The communication means 410 of the navigation module may send a request for obtaining navigation data representative of geographical position of the vehicle and real-time traffic, from a navigation services provider with communicating the starting point S and destination point D. The communication means 410 of the navigation module can receive such navigation data. In a complementary way, the navigation services provider comprises communication means for receiving the request and for transmitting the navigation data.

All received data by the communication means 410 can be supplied to and from the computing or control means 430 embedded in the vehicle or to cloud computing services. Preferably, the communication means 410 and/or the computing or control means 430 have at least one memory to store the collected data.

The communication means 410 of the navigation module may send a request to the cloud for obtaining the plurality of routes with communicating the starting point S and destination point D. In this case, cloud computing services can determine the plurality of routes. The communication means 410 of the navigation module may receive the determined plurality of routes and information about the travelling time. In a complementary way, the cloud comprises communication means for receiving the request and possibly for transmitting the determined plurality of routes.

Alternatively, computing or control means 430 embedded in the vehicle, for example part of the on-board computer of the vehicle, or through the application of the smartphone, are configured to determine the plurality of routes based on the navigation data, the starting point S and the destination point D. In that case, the control means 430 are configured to receive data from the communication means 410 and/or from the user interface.

The communication means 410 may be also adapted to communicate with other vehicles comprising a system according to the invention, for example via internet or by other means, so that data can be exchanged.

The communication means 410 are further adapted to acquire or obtain air pollution forecast data and possibly weather forecast data for the plurality of routes. In particular, the communication means 410 may be adapted to obtain air pollution forecast data for example in form of the pollution map data, which is indicative of most current air pollution levels within a specified area and a forecast for air pollution levels in the future, from a data supplier.

It is for example, communication means 410 of the vehicle's on-board computer or of the smartphone. The communication means 410 may send a request for obtaining air pollution forecast data, such as the pollution map, from a data supplier of air pollution forecast data with communicating the specified area. The communication means 410 can receive such air pollution forecast data. In a complementary way, the data supplier comprises communication means for receiving the request and for transmitting the air pollution forecast data.

Preferably, the system further comprises pollution sensing means 420 adapted to be mounted on a vehicle and to be configured to provide real-time pollution measurement data, indicative of pollution levels within the vehicle cabin and/or outside the vehicle's cabin, in its immediate vicinity.

Possibly, the communication means 410 may send a request for obtaining weather forecast data from a data supplier with communicating the specified area. The communication means 410 can receive the weather forecast data. In a complementary way, the data supplier comprises communication means for receiving the request and for transmitting the weather forecast data. Preferably, the communication means 410 are configured to obtain weather forecast data which is at least partly obtained from the vehicle's system information source comprising at least one from the group of: wipers speed, lighting operation, braking efficiency.

The communication means 410 of the vehicle's on-board computer or of the portable communication device or mobile terminal such as the smartphone may transmit the air pollution forecast data received from the data supplier and/or the pollution measurement data from the sensing means 420 and possibly the weather forecast data received from the data supplier and/or from the vehicle's system information source, to the cloud. In a complementary way, the cloud comprises communication means for receiving these data. Thus, the cloud can receive and store from different vehicles, so that those data can be used by different users. The data may also be used internally to supplement available data.

Alternatively, the communication means 410 may transmit the pollution and possibly weather forecast data to the computing or control means 430 embedded in the vehicle, for example part of the on-board computer of the vehicle, or through the application of the smartphone. The control means 430 are configured to receive this data from the communication means 410.

Further, the communication means 410 and/or the control means 430 are adapted to acquire or obtain user preference information from the user. This may be done by integration with any known input means, which might be a part of output means 440, e.g. by means of a touch screen of the vehicle entertainment system or through an application on the smart phone.

More precisely, the user can input preference information through the user interface such as the touch screen of the vehicle's entertainment system or of the smartphone. The communication means 410, for example of the vehicle's entertainment system or through an application on the mobile terminal, are configured to receive information from the user interface.

In one option, the communication means 410 can then transmit the preference information to the computing or control means 430 embedded in the vehicle, for example part of the on-board computer of the vehicle, or through the application of the smartphone. In another option, the communication means 410 are configured to transmit the preference information to cloud computing services.

The control means 430 embedded in the vehicle or the cloud computing services are configured to receive data from the communication means 410, and possibly from pollution sensing means 420 or from data supplier. The control means 430 can comprise memory for storing the obtained data.

The computing or control means 430 embedded in the vehicle or the cloud computing services are further configured to determine a first route score, based on user preference information, for each of the plurality of routes without taking into account the weather forecast data and the air pollution forecast data so that a first group of routes is obtained. For that purpose, the computing or control means 430 embedded in the vehicle or the cloud computing services may be adapted to compare the plurality of routes each other based on the user preference information and advantageously based on real-time information provided by the communication means 410 of the system.

The computing or control means 430 embedded in the vehicle or the cloud computing services are configured to calculate the average air quality index, based on air quality indexes received from the communication means 410 of the system.

The computing or control means 430 may further determine the travelling time of each route of the determined first group of routes or possibly receive the travelling time information from the communication means 410, for the whole trip and/or when the vehicle operates in a fresh air mode.

The computing or control means 430 are configured to calculate exposure factor based on the average air quality index and the travelling time for each of the first group of routes applying the formula (a) or during a fresh air mode applying the formula (b).

The computing or control means 430 embedded in the vehicle or the cloud computing services are also configured to determine a second route score, based on user preference information, for each of the first group of routes with taking into account the air pollution forecast data and the calculated exposure factors, and possibly the weather forecast data, so that a second group of routes is obtained.

The computing or control means 430 may be configured to compare the calculated exposure factors each other to determine the lowest.

Preferably, the computing or control means 430 are configured to calculate and compare the additional time of each route of the first group of routes to the fastest route.

The computing or control means 430 may be configured to compare air quality indexes acquired at the different measuring points along each route to a first threshold for instance 25, and/or to a second threshold for instance 50 or 75.

The computing or control means 430 may be configured to calculate the exposure time while the air quality index is less than or equal to a first threshold, and/or the exposure time while the air quality index is greater than the second threshold.

The computing or control means 430 may be configured to compare the calculated exposure times in order to determine the route with more exposure time with air quality index below or equal to the first threshold, and possibly less exposure time with air quality index above the second threshold.

Additionally, the control means 430 may be adapted to acquire vehicle parameters data for instance relating to vehicle air quality system components. The vehicle air quality system parameters may be selected from a group comprising: air mass flow through said installation, recirculation rate of air mass flow, filter parameters, separation of the cabin. The filter parameters may comprise filter separation efficiency and/or active area of the filter.

The control means 430 may also be configured to determine locations along the routes where the air outside is less polluted and forecast opening areas of the recycling flap. The detection module when provided can detect at least one parameter inside the car such as the temperature, carbon dioxide concentration $CO_2$ level, humidity level, and the number of passengers, and can be adapted to transmit data relating to the at least one parameter to the control means 430 of the system. Then, the control means 430 of the system may be adapted to receive inputs from the detection module and to calculate the level of carbon dioxide concentration $CO_2$ level or humidity level, inside the vehicle cabin at any time based on the initial value and to compare the obtained value to the at least one threshold limit, for defining where the recycling flap has to switch between open or close position.

The control means 430 may also be adapted to estimate weight of the filter using the obtained data and to match the estimated weight with a predefined clogging level. The control means 430 can comprise memory for storing the obtained data, as well as the estimate function data and predefined clogging levels associated with estimated weight. The memory can also store calibration parameters and instructions, as well as information about the last filter replacement or first installation.

The computing or control means 430 embedded in the vehicle or the cloud computing services may further be configured to compare the different routes based on the parameters or criteria previously described and to select a first route candidate R1 with optimal route score from the first group of routes and a second route candidate R2 with optimal route score from the second group of routes.

The computing or control means 430 embedded in the vehicle or the cloud computing services may then be configured to send an output command to the output means 440, with communicating information about the first route candidate R1 and the second route candidate R2.

The output means 440 are configured to receive such information and to output a first route candidate R1 and a second route candidate R2. The output means 440 can be carried out in form of visual output device like the touch screen of the vehicle's on-board computer and/or of the smartphone. In this case, the output means 440 and the input means can be the same.

Thanks to an anticipation model using weather, industrial pollution and traffic forecast based on machine learning model, the data could be adjusted for the coming kilometers in real time thanks to on-board sensor.

In another option the system can be considered as the whole set including the on-board means of the vehicle and/or the mobile terminal as well as the cloud and/or the navigation data provider or data supplier or a remote server.

It is understood that embodiments of the present invention can be carried out in form of hardware, software or a combination of both. Any such software can be stored in the form of a volatile or non-volatile storage, for example a storage like a ROM, whether erasable or rewritable or not, or in the form of memory such as, for example RAM, memory chips, device or integrated circuits or an optically or magnetically readable medium such as, for example, a CD, DVD, magnetic disk or magnetic tape. It is understood that the storage devices and storage media are embodiments of machine-readable storage that are suitable for storing program or programs that, when executed, implement embodiments of the present invention. Accordingly, embodiments provide a program comprising code for implementing a system or method as described and a machine readable storage storing such a program. Further, embodiments of the present invention may be conveyed electronically via any medium such as a communication signal carried over a wired or wireless connection and embodiments suitably encompass the same.

Other variations to the disclosed embodiments can be understood and effected by those skilled in the art in practicing the claimed invention, from a study of drawings, the disclosure, and the appended claims. The mere fact that certain measures are recited in mutually different dependent claims does not indicate that a combination of these measures cannot be used to the advantage.

In conclusion, thanks to the use of pollution map and traffic estimation, the method as previously described allows to propose a clean route on demand of the user or automatically. Further, the data can be adjusted for the coming miles in real time thanks to on-board sensor or to the updated information coming from pollution map.

**Claims**

1. A computer-implemented method for determining a route, in particular for a vehicle, comprising steps of:

- acquiring navigation data, a starting point (S) and a destination point (D) and determining (110) from navigation data a plurality of routes between the starting point (S) and the destination point (D) and the travelling time (dT; dT_FA) for each of the plurality of routes;
- acquiring (130) air pollution forecast data including air quality indexes for the plurality of routes;
- acquiring (140) user preference information;
- determining (150) a first route score, based on user preference information, for each of the plurality of routes without taking into account the air pollution forecast data, so that a first ranked group of routes is obtained;
- calculating (160a) exposure factor (E; E_FA) based on the average air quality index (AQI; AQI_FA) and the travelling time (dT; dT_FA) for each route of said first group of routes;
- determining (160) a second route score, based on user preference information, for each route of said first group of routes with taking into account the air pollution forecast data and the calculated exposure factor (E; E_FA), so that a second ranked group of routes is obtained;
- selecting and outputting (170) a first route candidate (R1) and a second route candidate (R2), wherein the first route candidate (R1) is the candidate with optimal first route score from said first group of routes and the second route candidate (R2) is the candidate with optimal second route score from said second group of routes.

2. A method according to claim 1, configured to be implemented by a system including an on-board computer of the vehicle and/or an information system source of the vehicle and/or an entertainment system of the vehicle and/or a mobile terminal and/or a user interface and/or a navigation services provider and/or a data supplier and/or cloud computing services, said system including communication means (410), control means (430) and output means (440), wherein:

- the steps of acquiring navigation data, a starting point (S) and a destination point (D) and determining (110) a plurality of routes between the starting point (S) and the destination (D) are implemented by the communication means (410) and control means (430) of the system;
- the step of acquiring (130) air pollution forecast data including air quality indexes for the plurality of routes is implemented by the communication means (410) of the system;
- the step of acquiring (140) user preference information is implemented by the communication means (410) and/or control means (430) of the system;
- the step of determining (150) a first route score, based on user preference information, for each of the plurality of routes without taking into account the air pollution forecast data, so that a first ranked group of routes is obtained, is implemented by the communication means (410) and control means (430) of the system;
- the step of calculating (160a) exposure factor (E; E_FA) based on the average air quality index (AQI; AQI_FA) and the travelling time (dT; dT_FA) for each route of said first group of routes, is implemented by the control means (430) of the system;
- the step of determining (160) a second route score, based on user preference information, for each route of said first group of routes with taking into account the air pollution forecast data and the calculated exposure factor (E; E_FA), so that a second ranked group of routes is obtained, is implemented by the control means (430) of the system;
- the step of selecting a first route candidate (R1) and a second route candidate (R2), is implemented by the control means (430) of the system;
- the step of outputting (170) a first route candidate (R1) and a second route candidate (R2), is implemented by output means (440).

3. A method according to claim 1 or 2, wherein the exposure factor (E) is calculated applying the formula (a):

$$(a)\ E = AQI * dT$$

with

AQI being the average air quality index,
dT being the traveling time in minutes.

4. A method according to any of claims 1 to 3, wherein the second route candidate (R2) with optimal second route score is the candidate route with the minimal exposure factor (E; E_FA) from the second group of routes.

5. A method according to any of claims 1 to 3,

- comprising the step of calculating (160b) exposure time with average air quality index (AQI; AQI_FA) less than or equal to a first threshold for each of the first group of routes; and
- wherein the step of determining (160) a second route score for each of the first group of routes is implemented with taking into account the exposure time with average air quality index (AQI; AQI_FA) less than or equal to the first threshold calculated, so that said second group of routes is obtained.

6. A method according to claim 5, wherein the second route candidate (R2) with optimal second route score is the candidate route with the longest exposure time (dT_AQI≤25) with average air quality index (AQI; AQI_FA) less than or equal to the first threshold from said second group of routes.

7. A method according to claim 5, comprising steps of:

- calculating (160c) exposure time with average air quality index (AQI; AQI_FA) greater than a second threshold for each of the plurality of routes and/or
- calculating (160d) exposure time with average air quality index (AQI; AQI_FA) greater than the first threshold and less than or equal to the second threshold for each of the plurality of routes; and

wherein the step of determining (160) a second route score for each of said first group of routes is implemented with taking into account the exposure time (dT_AQI>50) with average air quality index (AQI;

AQI_FA) greater than the second threshold and/or the exposure time (dT_25 <AQI≤50) with average air quality index (AQI; AQI_FA) greater than the first threshold and less than or equal to the second threshold calculated, so that said second group of routes is obtained.

8. A method according to any preceding claim, wherein the vehicle comprises a heating and/or ventilating and/or air conditioning installation able to operate in a fresh air mode, wherein:

- calculating (160a) exposure factor (E_FA) is implemented based on the average air quality index and the travelling time (dT_FA) when said installation operates in the fresh air mode for each of said first group of routes, applying the formula (b):

$$(b)\ E\_FA = AQI\_FA * dT\_FA$$

with

AQI_FA being the average air quality index when said installation operates in the fresh air mode,
dT_FA being the traveling time when said installation operates in the fresh air mode in minutes.

9. A method according to any preceding claim, wherein the vehicle comprises a heating and/or ventilating and/or air conditioning installation including a filter, said method:

- comprising the step of estimating (160e) the filter clogging ($C_{filter}$) level, and wherein
- the step of determining (160) a second route score for each of said first group of routes is implemented with taking into account the estimated filter clogging ($C_{filter}$) level.

10. A method according to any preceding claim, wherein the air pollution forecast data is at least partly obtained from a pollution map from a data supplier and/or from vehicle's onboard pollution sensors.

11. A method according to any preceding claim, wherein the method is iterated based on elapsed time or based on a travelled distance.

12. A method according to any preceding claim, wherein the method is iterated for each update of air pollution forecast data.

13. A method according to any preceding claim, wherein the first and/or second route score is determined based on at least one criterion selected from the group: distance, duration, cost.

14. A system comprising:

- communication means (410) and control means (430) configured to:

acquire navigation data, starting point (S) and destination point (D),
determine a plurality of routes from navigation data between the starting point and destination point and determine the travelling time (dT; dT_FA) for each of the plurality of routes,
acquire air pollution forecast data including air quality indexes for the plurality of routes,
acquire user preference information;
determine a first route score, based on user preference information, for each of the plurality of routes without taking into account the air pollution forecast data so that a first ranked group of routes is obtained; and
calculate the average air quality index (AQI; AQI_FA) for each route of the first group of routes, and the exposure factor (E; E_FA) based on the average air quality index (AQI; AQI_FA) and the travelling time (dT; dT_FA) for each route of the first group of routes,
determine a second route score, based on user preference information, for each route of the first group of routes with taking into account the air pollution forecast data and the calculated exposure factor so that a second ranked group of routes is obtained,
select a first route candidate (R1) and a second route candidate (R2), wherein the first route candidate (R1) is the candidate with optimal route score from said first group of routes and the second route candidate (R2) is the candidate with optimal route score from said second group of routes;

- output means (440) configured to output the first route candidate (R1) and the second route candidate (R2).

15. A computer readable storage medium storing a program of instructions executable by a machine to perform a method according to any of claims 1 to 13.

# Fig.1

# Fig.2

|      | Ra   | Rb   | Rc   |
|------|------|------|------|
| AQI  | 45   | 30   | 40   |
| dT   | 60   | 80   | 70   |
| E    | 2700 | 2400 | 2800 |

# Fig.3

|              | Ra   | Rb   | Rc   |
|--------------|------|------|------|
| AQI          | 45   | 45   | 39   |
| dT           | 60   | 60   | 70   |
| E            | 2700 | 2700 | 2710 |
| dT_AQI≤25    | 36   | 0    | 17.5 |
| dT_AQI>50    | 24   | 0    | 0    |
| dT_25 <AQI≤50 | 0   | 45   | 52,5 |

# Fig.4

|        | Ra   | Rb   | Rc   |
|--------|------|------|------|
| AQI    | 30   | 50   | 50   |
| dT     | 60   | 55   | 60   |
| E      | 1800 | 2750 | 3000 |
| AQI_FA | 30   | 50   | 25   |
| dT_FA  | 30   | 20   | 30   |
| E_FA   | 900  | 1000 | 750  |

# Fig.5

## Fig.6

| Ra | Z1 | Z2 | Z3 | Z4 | Z5 | Z6 | Z7 |
|---|---|---|---|---|---|---|---|
| AFE | 100% | 96% | 95% | 91% | 90% | 86% | 85% |
| $C_{filter}$ | 0g | 4g | 5g | 9g | 10g | 14g | 15g |
| dT | 10 | 10 | 10 | 10 | 10 | 10 | 10 |
| AQI_FA | - | 50 | - | 50 | - | 50 | - |

## Fig.7

| Rb | Z1 | Z2 | Z3 | Z4 | Z5 |
|---|---|---|---|---|---|
| AFE | 100% | 98% | 98% | 92% | 90% |
| $C_{filter}$ | 0g | 2g | 2g | 8g | 10g |
| dT | 10 | 10 | 10 | 10 | 10 |
| AQI_FA | - | 25 | - | 75 | - |

## Fig.8

| Rc | Z1 | Z2 | Z3 | Z4 | Z5 |
|---|---|---|---|---|---|
| AFE | 100% | 94% | 92% | 90% | 90% |
| $C_{filter}$ | 0g | 6g | 8g | 10g | 10g |
| dT | 10 | 10 | 20 | 10 | 10 |
| AQI_FA | - | 75 | - | 25 | - |

**Fig.9**

**Fig.10**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/080053 A1 (RAKSHIT SARBAJIT K [IN]) 28 March 2013 (2013-03-28) | 1-4, 10-12, 14,15 | INV. G01C21/34 G01C21/36 |
| A | * figures 3,4 * | 5-7 | G01N33/00 |
| A | DE 10 2015 013596 A1 (DAIMLER AG [DE]) 28 April 2016 (2016-04-28) * figure 2 * * paragraphs [0005], [0006], [0007], [0023], [0025] * | 1-7, 10-15 | |
| A | PT 105 929 A (UNIV DO MINHO [PT]) 12 April 2013 (2013-04-12) * figure 11 * * page 10, line 8 - page 11, line 10 * | 1-7, 10-15 | |
| A | DE 10 2015 211776 A1 (BAYERISCHE MOTOREN WERKE AG [DE]) 29 December 2016 (2016-12-29) * figures 2,5 * * paragraphs [0044], [0045], [0051] * | 1-7, 10-15 | |
| A | US 2010/145569 A1 (BOURQUE FRANCIS [US] ET AL) 10 June 2010 (2010-06-10) * figure 5 * * paragraph [0033] * | 1-7, 10-15 | |

TECHNICAL FIELDS
SEARCHED      (IPC)

G01C
G06Q
G01N
F01N
B60H

~~The present search report has been drawn up for all claims~~

| Place of search | Date of completion of the search | Examiner |
|---|---|---|
| The Hague | 4 October 2019 | Berbil Bautista, L |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

............................................................

& : member of the same patent family, corresponding
    document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7, 10-15

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 19 16 9053

The Search Division considers that the present European patent application does not comply with the requirements of unity of invention and relates to several inventions or groups of inventions, namely:

```
1. claims: 1-7, 10-15

        claims 1-7 and 10-15 deal with comparing the air quality
        index to a threshold
                        ---

2. claim: 8

        claim 8 deals with a fresh air mode along the route
                        ---

3. claim: 9

        claim 9 deals with filter clogging along the route
                        ---
```

## ANNEX TO THE EUROPEAN SEARCH REPORT
## ON EUROPEAN PATENT APPLICATION NO.

EP 19 16 9053

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2019

| Patent document cited in search report | | Publication date | Patent family member(s) | | Publication date |
|---|---|---|---|---|---|
| US 2013080053 | A1 | 28-03-2013 | NONE | | |
| DE 102015013596 | A1 | 28-04-2016 | NONE | | |
| PT 105929 | A | 12-04-2013 | PT<br>WO | 105929 A<br>2013054310 A1 | 12-04-2013<br>18-04-2013 |
| DE 102015211776 | A1 | 29-12-2016 | NONE | | |
| US 2010145569 | A1 | 10-06-2010 | US<br>US | 2010145569 A1<br>2012191290 A1 | 10-06-2010<br>26-07-2012 |

EPO FORM P0459

For more details about this annex : see Official Journal of the European Patent Office, No. 12/82